(19) Europäisches Patentamt
European Patent Office
Office européen des brevets

(11) EP 0 624 553 B1

(12) EUROPEAN PATENT SPECIFICATION

(45) Date of publication and mention of the grant of the patent:
**04.08.1999 Bulletin 1999/31**

(51) Int. Cl.[6]: **C01G 23/053**, C01B 13/32, C09K 9/00, G03C 1/73

(21) Application number: **94107307.4**

(22) Date of filing: **10.05.1994**

(54) **Process for producing photochromic titanium oxide compounds**

Verfahren zur Herstellung von photochromen Verbindungen von Titanoxid

Procédé de préparation de composés d'oxyde de titane photochromique

(84) Designated Contracting States:
**DE FR GB IT**

(30) Priority: **12.05.1993 JP 11026593**

(43) Date of publication of application:
**17.11.1994 Bulletin 1994/46**

(73) Proprietor:
**SUMITOMO CHEMICAL COMPANY LIMITED**
**Osaka-shi, Osaka 541-0041 (JP)**

(72) Inventors:
- **Harakawa, Masaji**
  **Niihama-shi, Ehime-ken (JP)**
- **Yamamoto, Kazuo**
  **Niihama-shi, Ehime-ken (JP)**

(74) Representative:
**VOSSIUS & PARTNER**
**Siebertstrasse 4**
**81675 München (DE)**

(56) References cited:
**EP-A- 0 125 507**
**EP-A- 0 314 166**
**FR-A- 2 585 973**
**GB-A- 2 023 115**

- **PATENT ABSTRACTS OF JAPAN vol. 17, no. 285 (C-1066) 2 June 1993 & JP-A-05 017 152 (SUMITOMO CHEM CO) 26 January 1993**
- **JOURNAL OF THE AMERICAN CERAMIC SOCIETY., vol.67, no.6, June 1984, COLUMBUS US pages C113 - C116 BRUCE FEGLEY 'synthesis and characterization of monosized doped TiO2 powders'**

Remarks:
The file contains technical information submitted after the application was filed and not included in this specification

EP 0 624 553 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

This invention relates to a process for producing a titanium oxide compound suitable for use in the fields of coating, printing, resin pigmenting, cosmetics, optical memories, etc. which utilize a chromatic effect known as photochromism.

A unique function of reversibly changing color according to an intensity variation of light, that is photochromism, is exploited in a variety of applications, such as sunglasses. As chemical substances displaying photochromism, silver halides and spiropyran series organic compounds are already known. As to titanium oxide, it is known to exhibit photochromism in the presence of certain metals such as iron, chromium and manganese and such systems are disclosed in Japanese Unexamined Patent Publications (Kokai) Nos. 132811/1988, 364117/1992, 17152/1993 and 51209/1993, International Patent Publication WO-89/12084 and so on.

However, compositions prepared by calcining a simple powdery mixture of titanium oxide with a powder of photochromism-imparting metal or its salt such as sulfate, chloride, nitrate, acetate, etc., its oxide or its hydroxide, as proposed in the above patent literature, achieve only a weak level of photochromism. As a matter of fact, the photochromic effect of this level is diluted when other components are conjointly used as admixed therewith in various applications, with the result that significant color change is hardly observed. Thus, these compositions are not sufficiently satisfactory for practical purposes.

An object of this invention is to provide a process for producing a titanium oxide compound displaying an excellent photochromism which can be used in various fields.

This and other objects of the invention will become apparent from the following description.

The above object can be solved by a process as defined below which comprises mixing an organotitanium compound with a photochromism-imparting metal-containing organic compound in a specific fashion, subjecting the resultant mixture to cohydrolysis and calcining the hydrolyzate.

Thus the present invention provides a process for producing a photochromic titanium oxide compound which comprises the steps of

(i) dissolving at least one organotitanium compound and in an amount of 0.05 to 8.0 weight % calculated as the corresponding metal oxide relative to the amount of the resulting titanium oxide compound at least one organic compound containing at least one metal selected from the group consisting of sodium, iron, chromium, copper, nickel, vanadium, manganese, silicon, zinc, aluminum, cerium, cobalt, niobium, zirconium, molybdenum and silver in an organic solvent to form a homogeneous solution, and if desired, partially removing said organic solvent from the solution,
(ii) substantially simultaneously hydrolyzing the organotitanium compound and the metal-containing organic compound in the solution obtained in step (i),
(iii) separating the organic solvent from the resultant hydrolysate, and
(iv) calcining the hydrolyzate at about 500 to 750°C for a least 1 hour,
wherein said steps (ii) and (iii) may be carried out concurrently.

This invention is now described in detail.

As the organotitanium compound for use in this invention, any hydrolyzable organotitanium compounds can be employed. Among such organotitanium compounds are titanium alkoxides represented by the formula

$$Ti(OR)_nX_{4-n}$$

wherein R represents an alkyl, alkenyl or aryl group, X represents an organic residue or a halogen atom, and n represents an integer of 1 - 4, organotitanium chelate compounds, organotitanium complexes and the like.

Among said titanium alkoxides are alkoxides of titanium with alcohols having 1 - 17 carbon atoms. Particularly preferred for commercial purposes are titanium tetra-i-propoxide, titanium tetra-n-butoxide, titanium tetrakis(2-ethylhexyloxide), titanium tetrastearyloxide, and so on.

The organotitanium chelate compounds include compounds of titanium coordinated to the oxygen or nitrogen atoms of a chelating agent, such as titanium acetylacetonate, titanium ethyl acetoacetate, salicylaldehyde-ethyleneimine titanate, diacetone-alkoxytitanium, octylene glycoxytitanium, triethanolamine titanate and titanium lactate, among others.

Among said organotitanium complexes are compounds having cyclopentadiene or the like as the ligand, such as monocyclopentadienyltitanium trihalides, dicyclopentadienyltitanium dihalides, cyclopentadienyltitanium trimethoxide, cyclopentadienyltitanium triethoxide, cyclopentadienyltitanium tripropoxide and so on.

These organotitanium compounds can be used singly or in combination. Furthermore, these organotitanium compounds can be used in the form of condensation products, such as oligomers, e.g. dimers, trimers, tetramers, which

are obtained by partial hydrolysis of said titanium alkoxides, or in the form of compounds which are obtained by partial substitution with (a) certain organic residue(s) such as acetoxy group, obtainable by reacting a titanium alkoxide or an oligomer thereof mentioned above with acetic acid, and the use of such compounds is sometimes advantageous for controlling the moldability of the resulting titanium oxide compound when it is molded into a desired form. The above-mentioned oligomers of a titanium alkoxide, a titanium alkoxide partially substituted by acetoxy group, or an oligomer of a titanium alkoxide partially substituted by acetoxy group are known and readily available as described, for example, in "Metal Alkoxides" authored by D.C. Bradly et al., Academic Press (1978), and any of them which are soluble in the organic solvent to be used in the present invention can be advantageously used.

In accordance with this invention, for imparting photochromic properties, said organotitanium compound and at least one organic compound containing at least one metal selected from the group consisting of sodium, iron, chromium, copper, nickel, vanadium, manganese, silicon, zinc, aluminum, cerium, cobalt, niobium, zirconium, molybdenum and silver are dissolved in an organic solvent to obtain a homogeneous solution.

As the organic compound containing such a metal, those containing sodium, iron, copper or niobium are preferable, but any of metal-containing organic compounds which are hydrolyzable and soluble in the organic solvent capable of dissolving said organotitanium compound can be employed.

Thus, as typical examples of the metal-containing organic compound, the following compounds are usable.

(i) Metal alkoxides represented by the formula

$$M(OR')_x \text{ or } VO(OR')_x$$

wherein M represents sodium, iron, chromium, copper, nickel, vanadium, manganese, silicon, zinc, aluminum, cerium, cobalt, niobium, zirconium, molybdenum or silver, R' is an alkyl group having 1 to 5 carbon atoms and x is an integer of 1 to 5 and corresponds to the valence number of M;
(ii) metal complexes having β-diketone ligand(s) as coordinated to the above metal;
(iii) metal soaps represented by the formula

$$M(OCOC_nH_{2n+1})_x$$

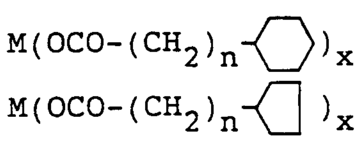

or

wherein M and x are as defined above and n is an integer of 1 to 18.
(iv) metallocenes such as ferrocene.

Examples of the metal alkoxide are:

- sodium methoxide, sodium ethoxide, sodium n-propoxide, sodium isopropoxide, sodium n-butoxide, sodium i-butoxide, sodium sec-butoxide, sodium t-butoxide;
- iron tri-methoxide, iron tri-ethoxide, iron tri-n-propoxide, iron tri-isopropoxide, iron tri-n-butoxide, iron tri-i-butoxide, iron tri-t-butoxide;
- tri-methoxy vanadyl , tri-ethoxy vanadyl , tri-n-propoxy vanadyl , tri-i-propoxy vanadyl , tri-n-butoxy vanadyl , tri-i-butoxy vanadyl , tri-t-butoxy vanadyl;
- manganese dimethoxide, manganese diethoxide, manganese di-n-propoxide, manganese di-i-propoxide;
- tetramethoxysilane, tetraethoxysilane, tetra-n-propoxysilane, tetra-i-propoxysilane, tetra-n-butoxysilane, tetra-i-butoxysilane, tetra-t-butoxysilane;
- zinc dimethoxide, zinc diethoxide, zinc di-n-propoxide, zinc di-i-propoxide, zinc di-n-butoxide, zinc di-i-butoxide, zinc di-t-butoxide;
- aluminum tri-methoxide, aluminum tri-ethoxide, aluminum tri-n-propoxide, aluminum tri-isopropoxide, aluminum tri-n-butoxide, aluminum tri-i-butoxide, aluminum tri-t-butoxide; and
- niobium penta-methoxide, niobium penta-ethoxide, niobium penta-n-propoxide, niobium penta-i-propoxide, niobium penta-n-butoxide, niobium penta-i-butoxide, niobium penta-t-butoxide.

Examples of the metal complexes having β-diketone ligand(s) are iron acetylacetonate, chromium acetylacetonate, copper acetylacetonate, vanadyl acetylacetonate, manganese acetylacetonate, zinc acetylacetonate, aluminum acetylacetonate, cobalt acetylacetonate, cerium acetylacetonate, etc.

Examples of the metal soaps are :

- sodium ethanoate, sodium propanoate, sodium butanoate, sodium hexanoate, sodium octanoate, sodium nonanoate, sodium decanoate, sodium undecanoate, sodium dodecanoate, sodium tetradecanoate, sodium hexadecanoate, sodium octadecanoate, sodium naphthenate;
- iron tri-ethanoate, iron tri-propanoate, iron tri-butanoate, iron tri-hexanoate, iron tri-octanoate, iron tri-nonanoate, iron tri-decanoate, iron tri-undecanoate, iron tri-dodecanoate, iron tri-tetradecanoate, iron tri-hexadecanoate, iron tri-octadecanoate, iron tri-naphthenate;
- chromium tri-ethanoate, chromium tri-propanoate, chromium tri-butanoate, chromium tri-hexanoate, chromium tri-octanoate, chromium tri-nonanoate, chromium tri-decanoate, chromium tri-undecanoate, chromium tri-dodecanoate, chromium tri-tetradecanoate, chromium tri-hexadecanoate, chromium tri-octadecanoate, chromium tri-naphthenate;
- copper di-ethanoate, copper di-propanoate, copper di-butanoate, copper di-hexanoate, copper di-octanoate, copper di-nonanoate, copper di-decanoate, copper di-undecanoate, copper di-dodecanoate, copper di-tetradecanoate, copper di-hexadecanoate, copper di-octadecanoate, copper di-naphthenate;
- nickel di-ethanoate, nickel di-propanoate, nickel di-butanoate, nickel di-hexanoate, nickel di-octanoate, nickel di-nonanoate, nickel di-decanoate, nickel di-undecanoate, nickel di-dodecanoate, nickel di-tetradecanoate, nickel di-hexadecanoate, nickel di-octadecanoate, nickel di-naphthenate;
- manganese di-ethanoate, manganese di-propanoate, manganese di-butanoate, manganese di-hexanoate, manganese di-octanoate, manganese di-nonanoate, manganese di-decanoate, manganese di-undecanoate, manganese di-dodecanoate, manganese di-tetradecanoate, manganese di-hexadecanoate, manganese di-octadecanoate, manganese di-naphthenate;
- aluminum tri-ethanoate, aluminum tri-propanoate, aluminum tri-butanoate, aluminum tri-hexanoate, aluminum tri-octanoate, aluminum tri-nonanoate, aluminum tri-decanoate, aluminum tri-undecanoate, aluminum tri-dodecanoate, aluminum tri-tetradecanoate, aluminum tri-hexadecanoate, aluminum tri-octadecanoate, aluminum tri-naphthenate;
- cobalt di-ethanoate, cobalt di-propanoate, cobalt di-butanoate, cobalt di-hexanoate, cobalt di-octanoate, cobalt di-nonanoate, cobalt di-decanoate, cobalt di-undecanoate, cobalt di-dodecanoate, cobalt di-tetradecanoate, cobalt di-hexadecanoate, cobalt di-octadecanoate, cobalt di-naphthenate; and
- silver ethanoate, silver propanoate, silver butanoate, silver hexanoate, silver octanoate, silver nonanoate, silver decanoate, silver undecanoate, silver dodecanoate, silver tetradecanoate, silver hexadecanoate, silver octadecanoate, silver naphthenate.

Particularly, metal alkoxides such as sodium ethoxide, β-diketone complexes such as iron acetylacetonate, metal soaps such as copper naphthenate, organometal complexes such as ferrocene and other metal chelate compounds can be mentioned.

The amount of such metal-containing organic compound is 0.05-8.0 weight %, preferably 0.1 to 5 weight %, calculated as the corresponding metal oxide, relative to the resulting final titanium oxide compound of the present invention (after calcination).

The organic solvents that can be employed are those capable of dissolving both of said organotitanium compound and said metal-containing organic compound, and it is preferable that such organic solvents have a melting point of 50 °C or lower (preferably -130 °C to 50 °C) and has a boiling point of 250 °C or lower (preferably room temperature to 250 °C) under a pressure of 1 atm.

Examples of useful organic solvent are:

- alcohols such as methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol, neopentyl alcohol, 1-hexanol, 2-methyl-1-pentanol, 4-methyl-2-pentanol, 2-ethyl-1-butanol, 1-heptanol, 2-heptanol, 3-heptanol, 1-octanol, 2-octanol, 2-ethyl-1-hexanol, 1-nonanol or 1-decanol;
- hydrocarbons such as pentane, 2-methylbutane, n-hexane, 2-methylpentane, 2,2-dimethylbutane, 2,3-dimethylbutane, heptane, octane, 2,2,3-trimethylpentane, 2,2,4-trimethylpentane, nonane, decane, dodecane, benzene, toluene, o-xylene, m-xylene, p-xylene, ethylbenzene, isopropylbenzene, 1,3,5-trimethylbenzene, tetralin, cyclopentane, methylcyclopentane, cyclohexane, methylcyclohexane or ethylcyclohexane;
- ethers such as diethyl ether, dipropyl ether, dibutyl ether, dihexyl ether, anisole, phenetole, butyl phenyl ether, dioxane, trioxane, furan, methylfuran, tetrahydrofuran, tetrahydropyran, 1,2-dimethoxyethane, 1,2-diethoxyethane, 1,2-dibutoxyethane, diethylene glycol dimethyl ether or diethylene glycol diethyl ether;

- ketones such as acetone, methyl ethyl ketone, 2-pentanone, 3-pentanone, 2-hexanone, methyl isobutyl ketone, 2-heptanone, 4-heptanone or diisobutyl ketone. These solvents can be used singly or at least two of them may be used in admixture.

Among them, there may be mentioned alcohols such as methanol, ethanol, 1- or 2-propanol, 1-, 2-, sec- or tert-butanol, etc., aromatic hydrocarbons such as benzene, toluene, xylene, etc., ethers such as diethyl ether, dioxane, tetrahydrofuran, etc., ketones such as acetone, methyl ethyl ketone, etc., aliphatic hydrocarbons such as pentane, hexane, octane, etc., and mixtures of two or more of such solvents.

The amount of the organic solvent is not so critical, but should be at least an amount sufficient to dissolve said organotitanium compound and metal-containing organic compound completely. It is generally recommended that the organotitanium compound be present in the solution at a concentration of about 0.1 to 90 weight %, preferably about 1 to 80 weight %.

Dissolution of said organotitanium compound and metal-containing organic compound can be effected by any known procedure such as the procedure employing a reaction tank equipped with a stirrer. There is no particular limitation on the manner of dissolving the two components. Thus, the procedure of dissolving one of said organotitanium compound and said metal-containing organic compound in the organic solvent in the first place and adding and dissolving the other in the resultant solution, the procedure of preparing respective solutions of the two compounds and blending the solutions, or the procedure of adding and dissolving both compounds in the organic solvent concurrently can be employed. For the preparation of a homogeneous solution, it is useful to dissolve the compound or compounds with stirring under heating and/or under reflux.

The solution (hereinafter sometimes referred to as "composition A") obtained by dissolving said organotitanium compound and metal-containing organic compound in said organic solvent is then contacted with water for substantially simultaneously hydrolyzing said organotitanium compound and metal-containing compound. The expression "to substantially simultaneously hydrolyze" as used herein means that the organotitanium compound and metal-containing organic compound are hydrolyzed in a parallel manner and, in this specification, this reaction is sometimes referred to briefly as "cohydrolysis".

The contacting of said composition A with water for this cohydrolysis can be carried out in a per se known manner. Thus, when water is used in the liquid state, water or an organic solvent such as one mentioned above (one capable of dissolving both of said organotitanium compound and said metal-containing organic compound) which contains water may be added to said composition A, or alternatively, said composition A may be added dropwise to water or said water-containing organic solvent. When water is used in the gaseous state, for example, said composition A containing a condensation product of organotitanium compound may be extruded in an atmosphere containing water vapor, or alternatively, a steam-containing nitrogen gas or a steam as such may, for example, be bubbled into said composition A. Furthermore, composition A can be subjected to said cohydrolysis after partial removal of the organic solvent by evaporation or distillation, or the organic solvent may be evaporated off after the cohydrolysis. As a further alternative, said cohydrolysis and removal of the organic solvent may be carried out concurrently.

The amount of water to be used is not critical. Generally, it is at least one mole, usually about 1 to 100 moles, per mole of the total amount of the organotitanium compound and the metal-containing organic compound present in said composition A. (The number of moles of the total amount of organotitanium compound and the metal-containing organic compound present in said composition A is a sum of the number of moles of organotitanium compound and the number of moles of the metal-containing organic compound present in said composition A.)

The temperature at which this cohydrolysis reaction is conducted is not critical, either, provided that it is lower than the decomposition temperatures of said organotitanium compound and metal-containing organic compound. Thus, this cohydrolysis reaction can be carried out at room temperature or, from productivity considerations, is carried out under heating.

Although depending on the kind of the organotitanium compound and metal-containing organic compound used, concentrations thereof, cohydrolysis temperature, stirring conditions and the like, the water used for the cohydrolysis usually reacts with the organotitanium compound and metal-containing organic compound in a short period of time, and therefore the degree of hydrolysis depends on the amount of water used for the cohydrolysis reaction. Thus, the amount of water to be used for the cohydrolysis may be suitably determined by evaluating or monitoring the photochromic properties of the product obtained by conducting the cohydrolysis under particular reaction conditions (e.g., manner how composition A is contacted with water, cohydrolysis temperature, etc.) and calcining the resulting hydrolysate. As mentioned above, if water is used in an amount of at least one mole, per mole of the total amount of the organotitanium compound and metal-containing organic compound, the desired titanium oxide compound having sufficient photochromic properties is usually obtained.

Removal of the organic solvent from composition A in step (i) or from the hydrolyzate in step (iv) can be conducted by conventional methods such as evaporation, spray drying or drum drying to be described below.

There is no particular limitation on the form of the hydrolyzate that can be produced by said cohydrolysis and removal of the organic solvent (the hydrolyzate thus obtained will sometimes be referred to merely as "hydrolyzate"). Thus, the hydrolyzate can be provided in a variety of forms suited for intended uses, e.g., in the form of powder, plate, sheet, flake, fiber or film, or in the form of coating layer prepared by coating a substrate or powder with the hydrolyzate.

Referring to the hydrolysate in the form of such powder, plate, sheet or flake, for instance, these are suitable for coating, printing, filler, cosmetics and other applications. The hydrolysate in the form of fiber can be used in the fields of construction and ornamentation. The hydrolysate in the form of coating layer as formed by coating on various surfaces are suitable for shutters, sensors and other uses.

For the production of such diverse forms of the hydrolyzate, conventional processing technologies can be used. The powder form hydrolysate, for instance, can be produced by contacting composition A with water for conducting cohydrolysis in the liquid phase and removing the water and organic solvent from the reaction system, or by contacting composition A with water vapor contained in the air or other gas using a spray drying or drum drying equipment for conducting cohydrolysis and removal of the organic solvent.

The process which can be employed for the production of the hydrolysate in the form of plate, sheet or flake may comprise the steps of coating a drum of a drum flaking machine or a belt-like substrate with composition A to form a wet coating film thereon, then, after partial evaporation of the organic solvent, contacting the partly dried film with water contained in the air while evaporating the remaining organic solvent, and then peeling or scraping the resulting dry film off. In this connection, the desired form of the hydrolyzate can be obtained by optimizing the concentration of the organotitanium compound, the heating temperature for evaporation of the organic solvent, the moisture content of the gas phase for hydrolysis and the temperature of hydrolysis.

The fiber form hydrolysate can be produced as follows. First, spinnability is imparted to compositon A, for example, by using an oligomer of an organotitanium compound and a metal-containing organic compound in the production of composition A, or by adding an organic polymer such as polyethylene glycol, polypropylene glycol or polyvinylpyrrolidone to composition A, or by subjecting composition A to a partial cohydrolysis to convert the titanium alkoxide to its oligomers as described in "Metal Alkoxides" authored by D.C. Bradly et al., Academic Press (1978). Then, composition A having spinnable properties is subjected to spinning with use of a dry-spinning apparatus or by centrifugal spinning or air-jet spinning and, then, to cohydrolysis.

When the hydrolysate is in the form of a coating layer as formed on a substrate surface, the substrate is dipped in or coated with composition A to form on the surface thereof a wet coating film, which is then subjected to cohydrolysis and evaporation of the solvent.

The hydrolyzate thus processed into the desired form is then calcined at a temperature of about 500 to about 750°C, preferably 550 to 700°C, generally for at least about 30 minutes, particularly at least about 1 hour, preferably about 1 to 3 hours, to thereby provide a photochromic titanium oxide compound in the desired form.

This titanium oxide compound is put to the intended use either as it is or, if required, after being subjected to neutralization, pulverization, milling, sieving and/or surface treatment.

The photochromic titanium oxide compound of this invention has or retains a color difference $\Delta E$ of at least 10, which is sufficient to permit a distinctly recognizable color change due to photochromism, even when used as admixed or diluted with other substances and is, therefore, of great commercial value.

As described above in detail, the process of the invention enables the production of a titanium oxide compound capable of displaying excellent photochromic properties in a variety of forms such as powders, plates, sheets, flakes, fibers, coating layers and so on.

The flaky titanium oxide compound obtainable by the process of this invention, of course, has the functions or properties, which are shared by the conventional flaky titanium oxides, such as whiteness or hiding power, or ability to take a laminar orientation of layers or adhesion associated with their shape or form. Moreover, the flaky titanium oxide compound of this invention, particularly those containing certain metals such as iron oxide, when used for cosmetics such as foundation, overcomes the disadvantage of the conventional cosmetics that the whiteness of the makeup is excessively accentuated upon exposure to brilliant sunlight. Thus, in response to the sunlight, the color of the skin made up indoors with a foundation cosmetic containing said titanium oxide compound of this invention quickly changes due to its excellent photochromic properties, and therefore the whiteness of the makeup which otherwise might be excessively high is rendered not so conspicuous even in the open air. Therefore, titanium oxide compound of the invention can provide natural-looking makeup cosmetics. When the titanium oxide compound of this invention is applied to signboards or displays, for instance, its whiteness changes according to the level of illumination, so that the whiteness becomes to match with the surrounding environment. The titanium oxide compound of this invention thus finds use in cosmetics, paints, filler, etc., and is of great industrial value.

EXAMPLES

The following examples are intended to illustrate this invention in further detail.

The evaluation of photochromism, as achieved by the present invention, was carried out by allowing to stand in the dark for at least 12 hours the product in the form of a powder, granule, plate or flake obtained in examples by the process of this invention and in comparison examples, then irradiating it with ultraviolet light at the intensity of 2 mW/cm$^2$ for 1 hour, determining the chromaticities before and after UV irradiation with a color difference meter (Model "Z-1001DP": product of Nippon Denshoku Kogyo Kabushiki Kaisha) and calculating the color difference ($\Delta E$ according to CIELAB standard colorimetric system) according to the following equation.

$$\Delta E = \text{(chromaticity before UV irradiation)} - \text{(chromaticity after UV irradiation)}$$

The fading rate was calculated, according to the following equation, from the color difference between chromaticity after 1-hour ultraviolet light irradiation and chromaticity after 10 minutes of fading in the dark following the 1-hour UV irradiation.

$$\text{Fading rate (\%)} = [\{(\text{Chromaticity after 1-hr UV irradiation}) - (\text{Chromaticity after 10-min fading in the dark})\} / (\text{Chromaticity after 1-hr UV irradiation})] \times 100$$

Example 1

A 3-necked separable flask of 300 ml capacity was charged with 32.0 g of titanium tetra-isopropoxide, 0.045 g of iron tri-isopropoxide and 114 g of isopropyl alcohol, and the mixture was stirred at room temperature for 1 hour.

After complete dissolution of the iron tri-isopropoxide, a solution prepared by dissolving 8.1 g of water in 154 g of isopropyl alcohol was added dropwise at room temperature over a period of 30 minutes to conduct cohydrolysis.

The reaction mixture was then heated at 90 °C in an oil bath to evaporate the solvent isopropyl alcohol, thereby giving a powder of the hydrolyzate.

This powder was placed in a porcelain crucible and heated in an electric furnace at a rate of 150°C/hr to 560°C and maintained at 560°C for 1 hour.

The photochromic properties of the resultant titanium oxide compound powder were then evaluated. $\Delta E$ = 29; fading rate = 30%.

Examples 2 - 4

Following the procedure of Example 1 and using the organotitanium compound, metal-containing organic compound, organic solvent, and organic solvent containing water for hydrolysis as shown in Table 1 and also employing the calcining temperatures shown in Table 1, powder form titanium oxide compounds of the invention were produced, and then the photochromic properties of each powder were evaluated. The results are shown in Table 1.

Table 1

| Example | Cohydrolysis (g) | | | | Calcining temperature (°C) | $\Delta E$ | Fading rate (%) |
|---|---|---|---|---|---|---|---|
| | Starting compounds | | Water-organic solvent system for hydrolysis | | | | |
| 2 | $Ti(Oi\text{-}C_3H_7)_4$ | 32 | $H_2O$ | 9.1 | 680 | 14 | 37 |
| | $Fe(AcAc)_3$* | 0.102 | $i\text{-}C_3H_7OH$ | 150 | | | |
| | $NaOC_2H_5$ | 0.719 | | | | | |
| | $i\text{-}C_3H_7OH$ | 30 | | | | | |
| 3 | $Ti(Oi\text{-}C_3H_7)_4$ | 32 | $H_2O$ | 9.1 | 600 | 13 | 5 |
| | Copper Naphthenate | 0.367 | $i\text{-}C_3H_7OH$ | 81 | | | |
| | $NaOC_2H_5$ | 0.301 | | | | | |
| | $i\text{-}C_3H_7OH$ | 100 | | | | | |

Table 1 (continued)

| Example | Cohydrolysis (g) | | | | Calcining temperature (°C) | ΔE | Fading rate (%) |
|---|---|---|---|---|---|---|---|
| | Starting compounds | | Water-organic solvent system for hydrolysis | | | | |
| 4 | $Ti(Oi\text{-}C_3H_7)_4$ | 32 | $H_2O$ | 9.1 | 600 | 15 | 7 |
| | $Nb(OCH_3)_3$ | 0.033 | $i\text{-}C_3H_7OH$ | 150 | | | |
| | $NaOc_2H_5$ | 0.301 | | | | | |
| | $i\text{-}C_3H_7OH$ | 100 | | | | | |
| In the table, $Fe(AcAc)_3$* represents iron acetylacetonate. | | | | | | | |

Example 5

A 3-necked separable flask of 300 ml capacity was charged with 22 g of toluene and 32 g of titanium tetra-isopropoxide and, then under stirring, a mixture of 1.15 g water, 28 g isopropyl alcohol and 30 g toluene was added dropwise to conduct condensation reaction to thereby dimerize the titanium tetra-isopropoxide. Then, 4.05 g of acetic acid was added dropwise to effect partial acetylation. Finally, 0.102 g of iron acetylacetonate and 0.719 g of sodium ethoxide were added and dissolved to provide a solution for the production of flakes.

This solution was applied to a drum flaker comprising a metal roll having a diameter of 150 mm and a rubber roll having a diameter of 100 mm, the two rolls rotating in contact with each other. The metal roll had a surface temperature of 90 °C and was rotating at 20 r.p.m. while the rubber roll was rotating in the opposite direction at 30 r.p.m. Thin liquid film of the solution was formed on the surface of the rubber roll by dipping a part of the rubber roll in the solution. Then, the liquid film was transferred to the surface of the metal roll through the point of contact of the two rolls. As the metal roll revolved (3/4 revolution), the solvent was partly evaporated for 0.75 second (1/4 revolution) and then the partly dried coating film was contacted with humidified air (temperature: 130 °C, dew point: 85 °C) for 1.5 seconds (1/2 revolution) while removing the remaining solvent from the coating film to dryness, whereby a dry cracked film was formed on the surface of the metal roll. Then, the film was scraped off with a scraper to provide flakes (hydrolysate).

These flakes were then heated in an electric furnace at a rate of 100°C/hr to 680°C and maintained at this temperature for 1 hour.

The resultant titanium oxide compound in the form of flakes showed a ΔE value of 13 and a fading rate of 45%.

Comparison Example 1

A 3-necked separable flask of 300 ml capacity was charged with 32.0 g of titanium tetraisopropoxide and 114 g of isopropyl alcohol.

To the mixture were added dropwise a solution prepared by dissolving 8.1 g of water in 154 g of isopropyl alcohol at room temperature over a period of 30 minutes to thereby effect hydrolysis reaction.

After completion of the hydrolysis, the reaction mixture was heated at 90 °C in an oil bath to remove the solvent isopropyl alcohol and recover a powder of the hydrolyzate.

This hydrolyzate powder was mixed with 31 g of water containing 0.20 g of ferric nitrate as dissolved therein and neutralized with 1.1 g of 4N aqueous solution of sodium hydroxide. The mixture was heated at 120 °C in an oil bath to evaporate the water and, then, heated in an electric furnace at a rate of 150°C/hr to 600°C and maintained at this temperature for 1 hour.

The resultant titanium oxide compound powder showed a ΔE value of 4 and a fading rate of 46%.

Comparison Example 2

A 3-necked separable flask of 300 ml capacity was charged with 22 g of toluene and 32 g of titanium tetra-isopropoxide, and a solution prepared by mixing 1.15 g water, 28 g isopropyl alcohol and 30 g toluene was added dropwise with stirring to conduct condensation reaction to thereby dimerize the titanium tetra-isopropoxide. Then, 4.05 g of acetic acid was added dropwise for partial acetylation to provide a solution for the production of flakes.

This solution was applied to the same drum flaker under the same condition as in Example 5, and flakes (hydrolysate) were thereby obtained.

Ten (10) g of the flakes were mixed with a solution prepared by dissolving 0.17 g of ferric nitrate in 23.2 g of

water, and after neutralization with 1.8 g of a 4N aqueous sodium hydroxide solution, the system was heated in an air bath to evaporate the water.

These flakes were then heated in an electric furnace at a rate of 150°C/hr to 600°C and maitained at this temperature for 1 hour. The resultant flakes of titanium oxide compound showed a ΔE value of 5 and a fading rate of 54%.

Comparison Example 3

Using a vibration mill, 9 g of ultrafine titania (tradename "P-25", product of Degussa) and 0.027 g of ultrafine iron oxide (tradename "Nanotite", product of Showa Denko Kabushiki Kaisha) were mixed and pulverized. The resultant powder was placed in a crucible and heated in an electric furnace at the rate of 150°C/hour to 560°C and maintained at this temperature for 1 hour. The photochromic properties of the resultant titanium oxide compound powder was then evaluated. ΔE = 3; fading rate = 67%.

Comparison Example 4

A 3-necked separable flask of 300 ml capacity was charged with 32.0 g of titanium tetra-isopropoxide and 114 g of isopropyl alcohol.

Then, a solution prepared by dissolving 8.1 g of water in 154 g of isopropyl alcohol was added dropwise over a period of 30 minutes at room temperature to thereby conduct hydrolysis reaction.

After completion of hydrolysis, the reaction mixture was heated at 90 °C in an oil bath to remove the solvent isopropyl alcohol and recover a powder of the hydrolyzate.

This powder was added to 30 g of an aqueous solution of 0.11 g of copper sulfate, and the whole system was heated at 120 °C in an oil bath with stirring to evaporate the water.

The resultant powder was heated in an electric furnace at the rate of 150°C/hour to 600°C and maintained at this temperature for 1 hour. The photochromic properties of the resultant titanium oxide compound powder was evaluated. ΔE = 3; fading rate = 10%.

Comparison Example 5

A 3-necked separable flask of 300 ml capacity was charged with 32.0 g of titanium tetraisopropoxide and 114 g of isopropyl alcohol.

Then, a solution prepared by dissolving 8.1 g of water in 154 g of isopropyl alcohol was added dropwise over a period of 30 minutes.

Then, the reaction system was heated at 90 °C in an oil bath to remove the solvent isopropyl alcohol and recover a powder of the hydrolyzate.

This powder was added to a solution prepared by dissolving 0.048 g of niobium chloride in 30g of ethanol. Then, 4.4 g of 1N aqueous solution of sodium hydroxide was added dropwise over a period of 1 minute at room temperature with stirring to hydrolyze the niobium chloride.

The mixture was heated in an oil bath to remove the solvent and, then, heated in an electric furnace at the rate of 150°C/hour to 560°C and maintained at this temperature for 1 hour. The photochromic properties of the resultant titanium oxide compound was evaluated. ΔE = 4; fading rate = 10%.

## Claims

**1.** A process for producing a photochromic titanium oxide compound, which comprises the steps of

(i) dissolving at least one organotitanium compound and in an amount of 0.05 to 8.0 weight %, calculated as the corresponding metal oxide, relative to the amount of the resulting titanium oxide compound, at least one organic compound containing at least one metal selected from the group consisting of sodium, iron, chromium, copper, nickel, vanadium, manganese, silicon, zinc, aluminum, cerium, cobalt, niobium, zirconium, molybdenum and silver in an organic solvent to form a homogeneous solution, and if desired, partially removing said organic solvent from the solution,
(ii) substantially simultaneously hydrolyzing the organotitanium compound and the metal-containing organic compound in the solution obtained in step (i),
(iii) separating the organic solvent from the resultant hydrolysate, and
(iv) calcining the hydrolyzate at about 500 to 750 C for at least 1 hour,
wherein said steps (ii) and (iii) may be carried out concurrently.

2. The process according to claim 1 wherein the organotitanium compound is a titanium alkoxide, an organotitanium chelate compound or an organotitanium complex.

3. The process according to claim 1 or 2 wherein the organotitanium compound is a titanium alkoxide.

4. The process according to any of claims 1 to 3 wherein the organotitanium compound is at least one member selected from the group consisting of titanium tetra-i-propoxide, titanium tetra-n-butoxide, titanium tetrakis(2-ethylhexyloxide), titanium tetrastearyloxide, titanium acetylacetonate, titanium ethyl acetoacetate, salicylaldehyde ethyleneimine titanate, diacetone alkoxy titanium, octylene glycoxy titanium, triethanolamine titanate, titanium lactate, monocyclopentadienyltitanium trihalides, dicyclopentadienyltitanium dihalides, cyclopentadienyltitanium trimethoxide, cyclopentadienyltitanium triethoxide and cyclopentadienyltitanium tripropoxide.

5. The process according to any of claims 1 to 4 wherein the organotitanium compound is a titanium alkoxide oligomer, a titanium alkoxide partially substituted by acetoxy group, or a titanium alkoxide oligomer partially substituted by acetoxy group.

6. The process according to any of claims 1 to 5 wherein the organic compound containing the metal is an organic compound containing sodium, iron, copper or niobium.

7. The process according to any of claims 1 to 6 wherein the organic compound containing at least one metal selected from the group consisting of sodium, iron, chromium, copper, nickel, vanadium, manganese, silicon, zinc, aluminum, cerium, cobalt, niobium, zirconium, molybdenum and silver is an alkoxide or chelate compound of said metal.

8. The process according to any of claims 1 to 7 wherein the organic solvent is selected from the group consisting of alcohols, hydrocarbons, ethers, ketones and mixtures thereof.

9. The process according to any of claims 1 to 8 wherein step (ii) is carried out by contacting the solution with water which is either in a liquid form or in a gaseous form.

10. The process according to any of claims 1 to 8 wherein step (ii) is carried out by contacting the solution with water which is in a liquid form.

11. The process according to any of claims 1 to 8 wherein step (ii) is carried out by contacting the solution with water which is in a gaseous form.

12. The process according to any of claims 1 to 11 wherein the hydrolysate is calcined at about 550 to 700 °C for 1 to 3 hours in step (iv).

13. A photochromic titanium oxide compound produceable by the process of any of claims 1 to 12, wherein said compound has a color difference $\Delta E$ of at least 10.

**Patentansprüche**

1. Verfahren zur Herstellung einer photochromen Titanoxidverbindung, umfassend die Schritte:

   (i) Lösen mindestens einer Organotitanverbindung in einer Menge von 0.05 bis 8.0 Gew.-%, berechnet als entsprechendes Metalloxid, bezogen auf die Menge der erhaltenen Titanoxidverbindung, und mindestens einer organischen Verbindung, die mindestens ein Metall, ausgewählt aus Natrium, Eisen, Chrom, Kupfer, Nickel, Vanadium, Mangan, Silicium, Zink, Aluminium, Cer, Cobalt, Niob, Zirkonium, Molybdän und Silber enthält, in einem organischen Lösungsmittel, zu einer homogenen Lösung, und falls gewünscht, teilweises Entfernen des organischen Lösungsmittels von der Lösung,
   (ii) im wesentlichen gleichzeitiges Hydrolysieren der Organotitanverbindung und der metallhaltigen organischen Verbindung in der in Schritt (i) erhaltenen Lösung,
   (iii) Abtrennen des organischen Lösungsmittels vom entstandenen Hydrolysat, und
   (iv) Brennen des Hydrolysats bei etwa 500 bis 750°C für mindestens 1 Stunde, wobei die Schritte (ii) und (iii) gleichzeitig durchgeführt werden können.

2. Verfahren nach Anspruch 1, wobei die Organotitanverbindung ein Titanalkoxid, eine Organotitanchelatverbindung

oder ein Organotitankomplex ist.

3. Verfahren nach Anspruch 1 oder 2, wobei die Organotitanverbindung ein Titanalkoxid ist.

4. Verfahren nach einem der Ansprüche 1 bis 3, wobei die Organotitanverbindung mindestens ein Vertreter, ausgewählt aus Titantetra-i-propoxid, Titantetra-n-butoxid, Titantetrakis(2-ethylhexyloxid), Titantetrastearyloxid, Titanacetylacetonat, Titanethylacetoacetat, Salicylaldehyd-Ethylenimintitanat, Diacetonalkoxytitan, Octylenglycoxytitan, Triethanolamintitanat, Titanlactat, Monocyclopentadienyltitantrihalogeniden, Dicyclopentadienyltitandihalogeniden, Cyclopentadienyltitantrimethoxid, Cyclopentadienyltitantriethoxid und Cyclopentadienyltitantripropoxid, ist.

5. Verfahren nach einem der Ansprüche 1 bis 4, wobei die Organotitanverbindung ein Titanalkoxidoligomer, ein teilweise mit einer Acetoxygruppe substituiertes Titanalkoxid oder ein teilweise mit einer Acetoxygruppe substituiertes Titanalkoxidoligomer ist.

6. Verfahren nach einem der Ansprüche 1 bis 5, wobei die das Metall enthaltende organische Verbindung eine Natrium, Eisen, Kupfer oder Niob enthaltende Verbindung ist.

7. Verfahren nach einem der Ansprüche 1 bis 6, wobei die organische Verbindung, die mindestens ein Metall, ausgewählt aus Natrium, Eisen, Chrom, Kupfer, Nikkel, Vanadium, Mangan, Silicium, Zink, Aluminium, Cer, Cobalt, Niob, Zirkonium, Molybdän und Silber, enthält, eine Alkoxid- oder Chelatverbindung des Metalls ist.

8. Verfahren nach einem der Ansprüche 1 bis 7, wobei das organische Lösungsmittel aus Alkoholen, Kohlenwasserstoffen, Ethern, Ketonen und Gemischen davon ausgewählt ist.

9. Verfahren nach einem der Ansprüche 1 bis 8, wobei Stufe (ii) durch Inkontaktbringen der Lösung mit Wasser, das entweder in flüssiger Form oder in gasförmiger Form ist, durchgeführt wird.

10. Verfahren nach einem der Ansprüche 1 bis 8, wobei Stufe (ii) durch Inkontaktbringen der Lösung mit Wasser, das in flüssiger Form ist, durchgeführt wird.

11. Verfahren nach einem der Ansprüche 1 bis 8, wobei Stufe (ii) durch Inkontaktbringen der Lösung mit Wasser, das in gasförmiger Form ist, durchgeführt wird.

12. Verfahren nach einem der Ansprüche 1 bis 11, wobei das Hydrolysat bei etwa 550 bis 700°C für 1 bis 3 Stunden in Stufe (iv) gebrannt wird.

13. Photochrome Titanoxidverbindung, herstellbar mit dem Verfahren nach einem der Ansprüche 1 bis 12, wobei die Verbindung einen Farbunterschied ΔE von mindestens 10 aufweist.

**Revendications**

1. Procédé de préparation d'un composé d'oxyde de titane photochromique, qui comprend les étapes de :

   (i) dissolution d'au moins un composé organique du titane et, en une quantité de 0,05 à 8,0% en poids calculée en tant que l'oxyde métallique correspondant, par rapport à la quantité du composé d'oxyde de titane résultant, d'au moins un composé organique contenant au moins un métal choisi dans le groupe consistant en sbdium, fer, chrome, cuivre, nickel, vanadium, manganèse, silicium, zinc, aluminium, cérium, cobalt, niobium, zirconium, molybdène et argent, dans un solvant organique de façon à former une solution homogène, et si cela est désiré, élimination partielle du dit solvant organique de la solution,
   (ii) hydrolyse de façon pratiquement simultanée du composé organique du titane et du composé organométallique dans la solution obtenue dans l'étape (i),
   (iii) séparation du solvant organique du prbduit d'hydrolyse résultant, et
   (iv) calcination du produit d'hydrolyse à une température d'environ 500 à 750°C pendant au moins une heure, dans lequel les dites étapes (ii) et (iii) peuvent être effectuées de façon simultanée.

2. Procédé suivant la revendication 1, dans lequel le composé organique du titane est un alcoolate de titane, un composé chélate organique du titane ou un complexe organique du titane.

3. Procédé suivant les revendications 1 ou 2, dans lequel le composé organique du titane est un alcoolate de titane.

4. Procédé suivant l'une quelconque des revendications 1 à 3, dans lequel le composé organique du titane est au moins un composé choisi dans le groupe consistant en tétra-i-propylate de titane, tétra-n-butylate de titane, tétrakis-(2-éthylhexylate) de titane, tétrastéarylate de titane, acétylacétonate de titane, éthyl acétoacétate de titane, titanate de salicylaldéhyde-éthylène imine, diacétone alcoxy titane, octylène glycoxy titane, titanate de triéthanolamine, lactate de titane, trihalogénures de monocyclopentadiényltitane, dihalogénures de dicyclopentadiényltitane, triméthylate de cyclopentadiényltitane, triéthylate de cyclopentadiényltitane et tripropylate de cyclopentadiényltitane.

5. Procédé suivant l'une quelconque des revendications 1 à 4, dans lequel le composé organique du titane est un oligomère d'alcoolate de titane, un alcoolate de titane partiellement substitué par un groupe acétoxy, ou un oligomère d'alcoolate de titane partiellement substitué par un groupe acétoxy.

6. Procédé suivant l'une quelconque des revendications 1 à 5, dans lequel le composé organométallique est un composé organique contenant du sodium, du fer, du cuivre ou du niobium.

7. Procédé suivant l'une quelconque des revendications 1 à 6, dans lequel le composé organique contenant au moins un métal choisi dans le groupe consistant en sodium, fer, chrome, cuivre, nickel, vanadium, manganèse, silicium, zinc, aluminium, cérum, cobalt, niobium, zirconium, molybdène et argent, est un alcoolate ou un chélate du dit métal.

8. Procédé suivant l'une quelconque des revendications 1 à 7, dans lequel le solvant organique est choisi dans le groupe consistant en alcools, hydrocarbures, éthers, cétones et leurs mélanges.

9. Procédé suivant l'une quelconque des revendications 1 à 8, dans lequel l'étape (ii) est conduite par mise en contact de la solution avec de l'eau qui est soit sous une forme liquide, soit sous une forme gazeuse.

10. Procédé suivant l'une quelconque des revendications 1 à 8, dans lequel l'étape (ii) est conduite par mise en contact de la solution avec de l'eau qui est sous une forme liquide.

11. Procédé suivant l'une quelconque des revendications 1 à 8, dans lequel l'étape (ii) est conduite par mise en contact de la solution avec de l'eau qui est sous une forme gazeuse.

12. Procédé suivant l'une quelconque des revendications 1 à 11, dans lequel le produit d'hydrolyse est calciné à une température d'environ 550 à 700°C pendant 1 à 3 heures dans l'étape (iv).

13. Composé d'oxyde de titane photochromique pouvant être produit par le procédé suivant l'une quelconque des revendications 1 à 12, dans lequel ledit composé a une différence de couleur $\Delta E$ d'au moins 10.